# EUROPEAN PATENT SPECIFICATION

(11) **EP 4 433 147 B1**
(45) Date of publication and mention of the grant of the patent: **06.05.2026**
(21) Application number: 22851037.6
(22) Date of filing: 28.12.2022
(51) Int. Cl.: A61N 1/02, A61N 1/04, A61N 1/36

(54) **ELECTRONIC CIRCUIT FOR DELIVERING BI-DIRECTIONAL ELECTRICAL STIMULATION**
ELEKTRONISCHE SCHALTUNG ZUR VERABREICHUNG VON BIDIREKTIONALER ELEKTRISCHER STIMULATION
CIRCUIT ÉLECTRONIQUE POUR FOURNIR UNE STIMULATION ÉLECTRIQUE BIDIRECTIONNELLE

(30) Priority: 28.12.2021 GB 202119050
(43) Date of publication of application: 25.09.2024
(73) Proprietor: Brainpatch Ltd, London SE11 5DP (GB)
(72) Inventor: VYSOKOV, Nickolai, London Kent TN15 6LJ (GB); TARASENKO, Illya, Wrotham Kent TN15 7BP (GB)
(74) Representative: Basck Limited
(86) International application number: PCT/IB2022/062840
(87) International publication number: WO 2023/126851

(56) References cited:
- WO-A1-2020/082135
- US-B2- 10 058 706
- US-B2- 8 755 904

## Description

### TECHNICAL FIELD

The present disclosure relates to electronic circuits for providing, when in use, one or more electrical stimulations. More particularly, the present disclosure relates to an apparatus for providing bi-directional stimuli to a user.

### BACKGROUND

It is known that electrical stimulation techniques have been used in various treatments to mitigate problems related to neuromuscular tissues, and also for improving physiological and mental well-being. Such stimulation techniques are beneficially implemented using apparatus comprising one or more modules, wherein a most relevant such module is a power or output stage, wherein the power or output stage is configured to deliver voltage or current signals of appropriate intensity to biological tissue, and wherein the signals can be applied to the biological tissue by at least one of non-invasive and invasive procedures.

Commonly known stimulation techniques include electrical muscle stimulation (EMS), temporal interference stimulation (TI), Russian electrical stimulation, neuromuscular electrical stimulation (NMES), functional electrical stimulation (FES), transcranial direct-current stimulation (tDCS), transcranial alternating-current stimulation (tACS), transcranial random noise stimulation (tRNS), transcutaneous electrical nerve stimulation (TENS) and more. Additionally, electrical stimulation can be combined with other stimulation methods, such as stimulation by applying magnetic fields, visual stimulation, audio stimulation and so forth. All of the above electrical stimulation methods refer to the same fundamental process, namely applying electricity to a living body to increase or decrease activity in a nervous system or in muscles of the living body. The aforesaid different names for commonly known stimulation techniques derive from applying an electrical current in mutually different ways, to mutually different parts of the living body, or for different purposes. Generally speaking, the different names reflect either the intended use of the electrical stimulation or the characteristics of the stimulation itself. For example, EMS and Russian electrical stimulation are both generally intended for athletic training, but Russian stimulation uses high frequency sinusoidal waveforms, whereas EMS typically uses lower frequency rectangular waveforms. As another example, TENS apparatus are typically used for pain relief, whereas NMES apparatus are used to retrain muscles after an injury, even though both TENS and NMES apparatus use mutually similar stimulation waveforms.

Furthermore, Transcranial direct current stimulation (tDCS) is a stimulation treatment, commonly used for cognitive enhancement, but also employed for treatment of various neurological disorders including Alzheimer's disease. tDCS uses direct electrical currents to stimulate specific parts of the brain. Specifically, in tDCS, a constant, low intensity current is passed between two electrodes placed over the head which modulates neuronal activity.

Furthermore, transcranial alternating current stimulation (tACS) is a stimulation treatment that uses alternating currents to stimulate specific parts of the brain. Specifically, in tACS, low-frequency currents (< 100 Hz) are applied; in the case of closed-loop phase-locked tACS, the exogenous oscillations are synchronized with the brain's endogenous frequency.

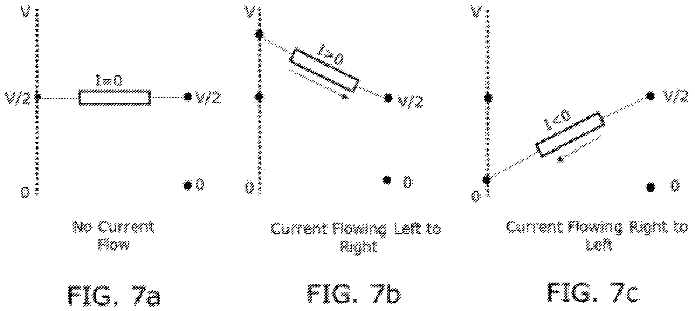

Known existing circuits, employed in the aforesaid commonly used stimulation techniques, function by fixing a voltage at a first electrode to a specific value, while varying a voltage on a second electrode (as shown in Fig. 7) relative to the first electrode. In a situation where a power source to the circuits supplies a total voltage of V volts, a voltage at the first electrode is fixed at a value of V/2 volts and a voltage at the second electrode is varied between 0 and V volt. When the voltage at the second (variable) electrode is also set to the value of V/2 volts (FIG 7a), there will be zero net potential difference between the first and second electrodes, thus resulting in zero current flow between the first and second electrodes. If the potential of the second (variable) electrode is set to V (FIG 7b), then there will be a net potential difference of V/2 volts between the first and second electrodes. This will result in the flow of current from the second (variable) electrode at higher potential to the first electrode at lower potential (namely, the electrode with fixed voltage). When the voltage at the second (variable) electrode is set to a voltage below the value of V/2 volts, the current will flow in reverse direction, namely from the first (fixed) electrode to the second (variable) electrode (as shown in Fig 7c). As the current is proportional to the potential difference, wherein in the existing circuit the maximum potential difference across the electrodes is limited to half of the maximum value of the power source voltage V, this means that there exists a problem of underutilization of the power source. In turn, this means that the power source must supply the circuit with at least twice the voltage, required to be applied across the first and second electrodes, as necessary to drive bi-directional current of a required magnitude.

A necessity to operate at higher voltages creates further difficulties and limitations in the design of circuits of stimulation devices. One difficulty arises from there being required a plurality of high-precision circuitry components (for example, operational amplifiers) that are rated to work at higher voltages, wherein a range of choice of such components supplied as integrated circuit (IC's) from suppliers is restricted. This means that simpler and cheaper circuits, that perform well at lower supply voltages, might be impossible to implement at higher voltages due to necessary components not being commercially available. In order to circumvent such a lack of commercially available integrated circuits, a need would arise to construct more complicated and expensive circuits altogether.

Another possible disadvantage of using higher supply voltages is to do with the size and portability of the apparatus, especially when the apparatus is configured in battery-operated implementations. To provide higher supply voltage, it would be necessary to use more/higher voltage batteries; implement bulky (inverter) circuitry that would convert lower voltage into higher voltage, or combine the two. The higher the necessary voltage, the more prominent these problems become.

Moreover, on top of these limitations, there are certain requirements stipulated by the standard organisations, such as ISO60601-2-10, that require accurate measurement of the current and/or voltage delivered at the electrodes to ensure that the delivered current matches the desired current, and that the voltage doesn't exceed threshold. While in standard stationary stimulators with a full positive and negative high voltage power supply current and voltage can easily be measured using simple operational amplifiers or instrumentation amplifiers, in portable stimulators with optimised power supply, this is no longer possible and requires special circuitry. US10058706 discloses a circuit arrangement for delivering stimuli to a user.

These and other issues may hinder the development of miniaturised and portable devices and apparatus, limiting for example the magnitude of maximum deliverable current, restricting the potential range of available wavefunctions, increasing the weight, size and cost of the circuits used, and thus ultimately limiting the range of potential applications of the devices and apparatus.

Therefore, despite advancements that have been made to date in the aforementioned electrical stimulation techniques and circuits used in apparatus implementing the techniques, there exists a need to redesign the circuits to provide both monophasic and biphasic stimulation with a wider range of available waveforms and frequencies, increased stimulation amplitude and improved miniaturisation and lower implementation cost.

### SUMMARY

The invention is defined by the independent claim 1. The present disclosure seeks to provide an easy-to-use current source arrangement for facilitating an increased (for example, maximum) bi-directional flow of current while using a single-rail power supply of as low voltage magnitude as practically possible.

According to a first aspect, the present disclosure provides a circuit arrangement for delivering stimuli to a user, wherein the circuit arrangement comprises a circuit including a pair of current pumps including a first current pump and a second current pump, and an inverting voltage mirror to provide an inverted signal to at least one of the first current pump and a second current pump to control its operation.

Embodiments of the disclosure are advantageous in terms of providing an easy-to-use circuit for providing bi-directional flow of current while using single-rail power supply while utilizing the full range of power supply voltage available for electrical stimulation in both forward and reverse current flow directions. Moreover, the circuit is based on simple electronic components such as operational amplifiers (op-amps), transistors, MOSFETs and so forth, thereby enabling the circuit to be cheaply manufactured, to have exceptional frequency range, to have good power efficiency and enable the circuit to be implemented in a spatially compact manner.

Optionally, in the circuit arrangement, the first current source and the second current source are located on same side of the electrodes that are in contact with a human body (i.e. constituting an electrical 'load') of the user.

Optionally, in the circuit arrangement, the inverting voltage mirror is configured to ensure a zero current flow in a rest state. By "zero" is meant substantially zero, for example less than 1% of a maximum current flow delivered by the circuit arrangement.

Optionally, the circuit arrangement is connected to a power source. More optionally, the power source includes at least one of a battery and a voltage boost circuit to increase voltage, for example, from 3.7V up to ~30-50V. Optionally, in the circuit arrangement, the inverting voltage mirror is configured to ensure a zero current flow in rest state.

Optionally, in the circuit arrangement, the inverting voltage mirror is located at other side of the load opposite to the first current pump and the second current pump.

Optionally, the circuit arrangement is configured to create a potential difference across the load required for supplying a predefined magnitude of stimuli to the user.

Optionally, the circuit arrangement is configured to deliver at least one of a transcranial direct current stimulation (tDCS), transcranial alternating current stimulation (tACS) and bipolar pulse stimulation.

Optionally, the circuit arrangement, when in operation, delivers electric stimuli to the user and receives signals from a portion of skin of the user, simultaneously. More optionally, the circuit arrangement includes a microprocessor configured to determine the parameters of stimulation. Optionally, one or more components of the circuit arrangement are arranged on an integrated circuitry microchip.

According to a second aspect, there is provided a non-claimed method for using the circuit arrangement of the first aspect to provide stimuli to a user via electrodes in electrical contact with the user.

Additional aspects, advantages, features and objects of the present disclosure would be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary embodiments of the disclosure are shown in the drawings. However, the present disclosure is not limited to specific methods and instrumentalities disclosed herein. Moreover, those skilled in the art will understand that the drawings are not to scale. Wherever possible, like elements have been indicated by identical numbers.

Embodiments of the present disclosure will now be described, by way of example only, with reference to the following diagrams wherein:
FIG. 1 is a block diagram of a circuit arrangement for delivering one or more electrical stimuli to a user, in accordance with an embodiment of the present disclosure;
FIG. 2a is a circuit diagram of a circuit arrangement, wherein the circuit includes two current sources, an inverting current mirror and the load;
FIG. 2b is a simplified circuit diagram of a circuit arrangement for delivering one or more electrical stimuli to a user, in accordance with an embodiment of the present disclosure;
FIG. 3 is a circuit diagram of a first current source present in the circuit arrangement, in accordance with an embodiment of the present disclosure;
FIG. 4 is a circuit diagram of a second current source present in the the circuit arrangement, in accordance with an embodiment of the present disclosure;
FIG. 5 is a circuit diagram of an inverting voltage mirror present in the the circuit arrangement, in accordance with an embodiment of the present disclosure;
FIGs. 6a and 6b are circuit diagrams of a voltage follower and a voltage subtractor present in the inverting voltage mirror, in accordance with an embodiment of the present disclosure;
FIG. 7 is a graphical representation of working of a conventionally used circuit in a stimulation arrangement; and
FIG. 8 is a graphical representation of working of a circuit present in the circuit arrangement, in accordance with an embodiment of the present disclosure.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item to which the arrow is pointing.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognise that other embodiments for carrying out or practising the present disclosure are also possible.

In an aspect, the present disclosure provides a circuit arrangement for delivering electrical stimulation/stimuli to a user, wherein the circuit arrangement comprises an electric circuit including a first current source, second current source and an inverting voltage mirror.

For example, the present disclosure provides a circuit arrangement for delivering stimuli to a user, wherein the circuit arrangement comprises a circuit including a pair of current pumps including a first current pump and a second current pump, and an inverting voltage mirror to provide an inverted signal to at least one of the first current pump and a second current pump to control its operation.

Throughout the present disclosure, the term *"user"* as used herein relates to any person (i.e., human being) using the aforesaid device. Optionally, the user may be a person having a certain physical or mental disorder such as epilepsy, a head injury, encephalitis, brain tumour, encephalopathy, memory related problems, sleep disorders, stroke, dementia, depression, etc. Alternatively, the user may be a person willing to achieve a specific state of mind, such as an enhanced concentration, relaxation, mental capabilities or, in general terms, enhanced performance for executing a task. For example, the user may be a person seeking relief from tinnitus.

Alternatively, the abovementioned circuitry may be used for delivering stimuli to muscles and/or tissues of animals, such as for scientific research, or for animal performance enhancement.

Throughout the present disclosure, the terms "stimuli", *"brain stimulus", "brain stimuli"* (plural of *"stimulus"*) or "stimulation" as used herein relates to an external electrical current or to a defined sequence or multiple sequences of electric current amplitudes between a pair of electrodes applied to the scalp and/or skin of other body parts of a user, in order to alter (referring to raising, lowering or otherwise modulating) levels of physiological or nervous activity in the brain/muscles or in the tissues spatially remote from the given user's brain/limbs that the current is able to reach. Optionally, the circuit arrangement is configured to deliver at least one of a transcranial direct current stimulation (tDCS), transcranial alternating current stimulation (tACS) and bipolar pulse stimulation.

Moreover, the circuit arrangement may be used to provide one or more stimuli to nerves, a vestibular system and so forth. Furthermore, in an example, stimuli applied to the user are analogue external electrical signals having a voltage in a range of, but not limited to, +/-1 milliVolt (mV) to +/-50 volts and having a current in a range of, but not limited to, +/-0.1 milliamperes (mA) to +/-20 milliamperes (mA).

Throughout the present disclosure, the terms *"current source"* and/or *"current pump"* as used herein relates to one or more components in an electric circuit or a circuit arrangement of components that drives electric current through a load, with a magnitude of the current being independent of the electric impedance of the load. Furthermore, the current source may include, but is not limited to, an independent current source that delivers a constant current and a dependent current source that delivers a current which is proportional to a control voltage or a control current.

Optionally, the current source may include constant current diodes, Zener diode current sources, transistor current sources, op-amp current sources, voltage regulator current sources, current mirrors or other current source circuits.

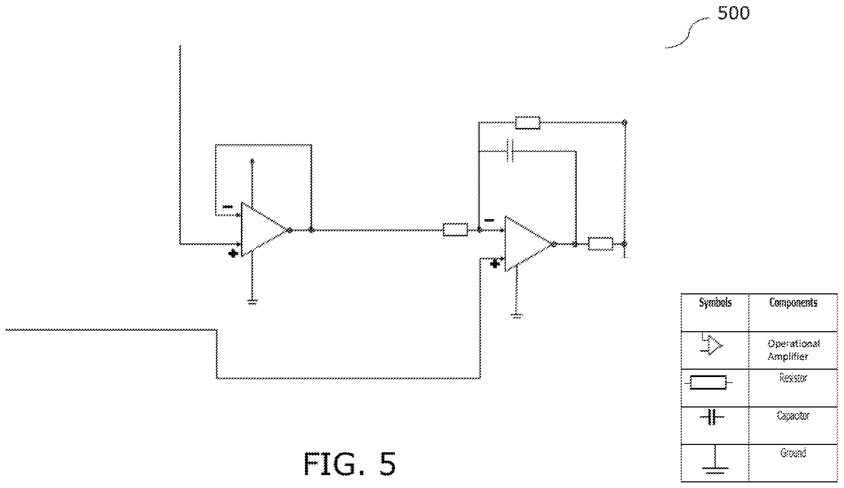

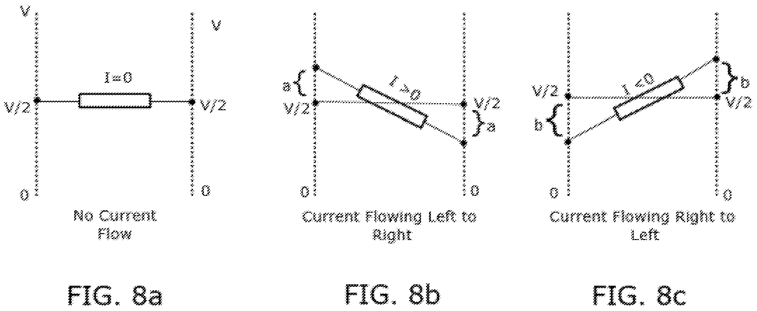

Throughout the present disclosure, the terms *"inverting voltage mirror",* as used herein, relates to a circuit that is designed to monitor and/or detect changes in voltage at an input terminal of the inverting voltage mirror (for example, a given end/node of the device circuit) and accordingly modulate the voltage at an output terminal of the inverting voltage mirror (for example a second end/node of the device circuit) (as shown in an exemplary implementation of FIG. 5). Specifically, the inverting voltage mirror modulates the voltage at the output terminal of the inverting voltage mirror in a direction that is opposite to the detected voltage change at the input terminal of the inverting voltage mirror. For example, in a situation where at the input the voltage is increased by the value 'a' with respect to a certain (reference) voltage (for example V/2, wherein V is the value of voltage, supplied by the power source, as schematically illustrated in Fig. 8b), the inverting voltage mirror will detect the positive change in voltage at the first end/node (input) and accordingly the inverting voltage mirror will decrease the voltage at the second end/node (output) by a value similar or equivalent to 'a' with respect to the same (reference) voltage (in this example V/2). Here 'a' can have any value between 0 volts and V/2 volts. This design will achieve a maximum potential difference of V/2+V/2=V or (-V/2)+(-V/2)=-V across the load. This means that the circuit is now capable of bi-directionally applying to the electrodes the potential difference, corresponding to the full voltage of the power supply, as illustrated in an exemplary implementation in FIG. 8.

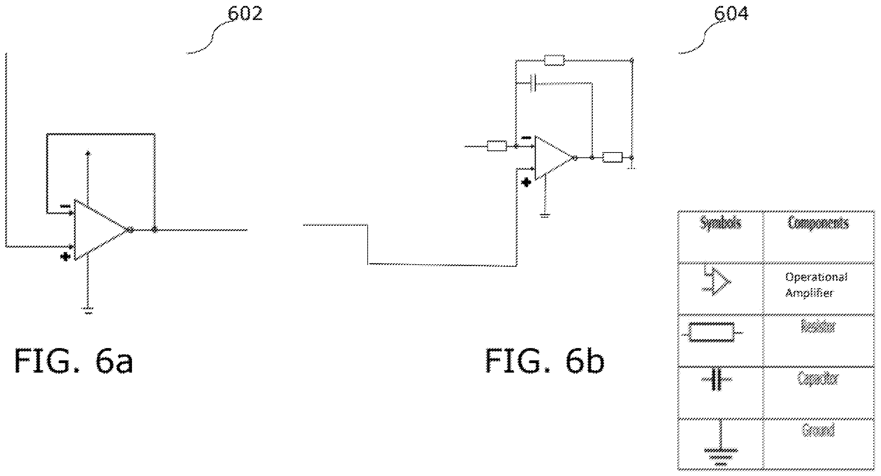

Optionally, the inverting voltage mirror includes a voltage follower (as shown in FIG. 6a) and a voltage subtractor (as shown in FIG. 6b). Specifically, the voltage follower is configured to monitor and/or detect voltage at the inverting voltage mirror input (first end/node of the device circuit). Furthermore, based on the voltage detected by the voltage follower, the voltage subtractor is configured to modulate the voltage at inverting voltage mirror output (the second end/node of the device circuit). Optionally, the first/second ends/nodes refer to points in the circuit at which the terminals of the load or the electrodes are located.

Furthermore, the inverting voltage mirror is a sub-circuit that is configured to be able to increase (for example, maximize) the potential difference across two ends/nodes of the primary circuit. The voltage being mirrored may be considered as the input of the sub-circuit. In some applications, high-gain high-speed operational amplifiers may be used to implement the inverting voltage mirror. Optionally, the voltage follower is also used to implement the inverting voltage mirror.

Throughout the present disclosure, the term *"electrode", "at least one electrode", "pair of electrodes"* or *"elongated electrode"* as used herein relates to one or more electrical conductors, with these conductors are manufactured from materials including, but not limited to, steel, platinum, sliver chloride-coated silver, carbon rubber, as well as hydrogels, silicone, sponges, foam or any absorbent with a conducting medium, where necessary to be placed between the conductors and the scalp or skin of other body parts. Furthermore, the electrodes, when applied to scalp or skin of other body parts of the user, and the body of the user are configured to work together as the 'load' for the circuit.

Moreover, the electrodes are optionally minimally invasive (such as needle electrodes or micro electrodes) or non-invasive type (such as surface electrodes), or optionally both. Furthermore, the electrodes may optionally include invasive electrodes such as strips, grids, depth electrodes and so forth.

In an embodiment, the circuit may beneficially include one current source and an inverting voltage mirror, arranged on opposite sides of a load. Specifically, on opposite ends of the pair of electrodes applied to scalp or skin of other body parts of the user. In one configuration of such a circuit arrangement, the inverting voltage mirror functions as a drain, enabling current flow from the current source to the drain through the load i.e., through the pair of electrodes applied to the scalp or skin of other body parts of a user. In another configuration of such a circuit arrangement, the inverting voltage mirror functions as a source, enabling current flow from the inverting current mirror into current source (here acting as a current drain).

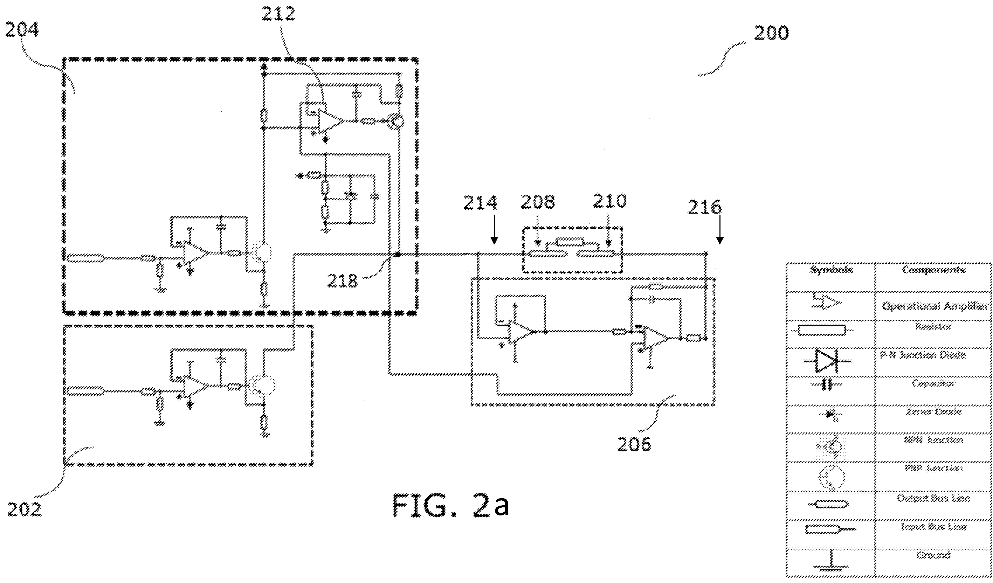

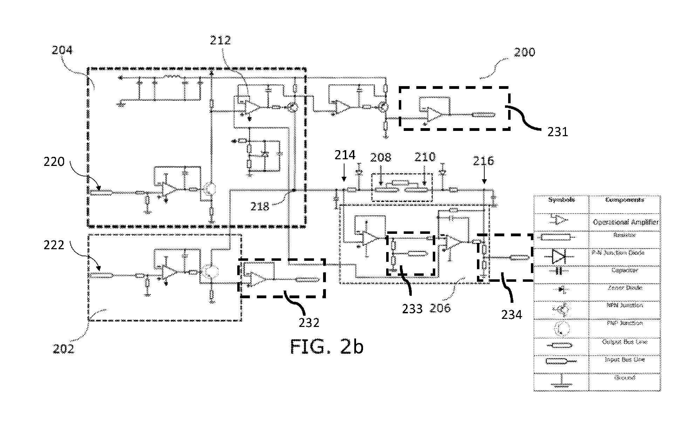

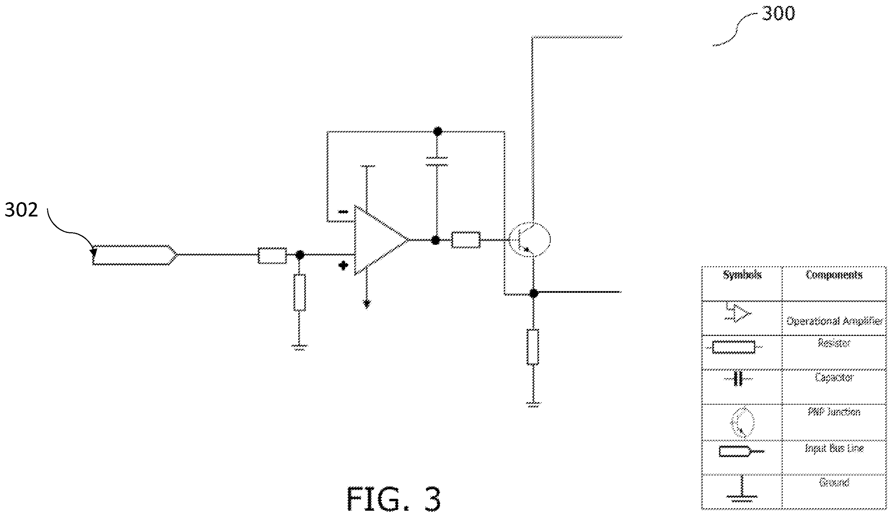

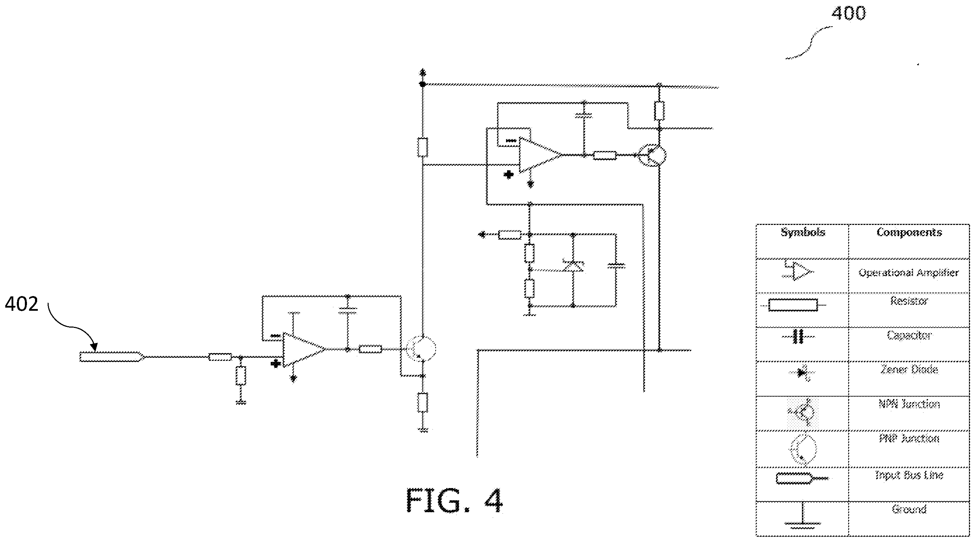

In another embodiment, the circuit may include two current sources including a first current source (FIG. 3) and a second current source (FIG. 4), and an inverting voltage mirror (FIG. 5) (as shown in an exemplary implementation in FIG. 2). Specifically, in this embodiment, the first current source and the second current source are located on one side of the load, and the inverting voltage mirror is located on the opposite sides of the load i.e., on the opposite sides of the pair of electrodes applied to the scalp or skin of other body parts of the user. Furthermore, the first current source and the second current source are connected in series. The second current source functions, when in use, as a source and the first current source functions as a drain. In a condition, when the first current source and the second current source are in equilibrium i.e., a situation in which the current drained by the first current source is the same as the current supplied by the second current source, the circuit arrangement will be in rest state and zero current will flow through the pair of electrodes applied to the scalp or skin of other body parts of the user i.e., through the load.

Furthermore, an imbalance across the first current source and the second current source is created to facilitate the flow of current through the load i.e., through the pair of electrodes applied to the scalp or skin of other body parts of the user. Specifically, in a situation in which the flow of current is required through the load in the forward direction (from two current sources into the inverting voltage mirror), the imbalance may be created in such a manner that the second current source provides more current than the first current source can sink, and the excess current 'over-flows' into the inverting voltage mirror through the load i.e., through the pair of electrodes, thus, supplying stimuli to the scalp or other body parts of the user.

Notably, to facilitate the flow of current in a reverse direction i.e., from the inverting voltage mirror into two current sources, the first current source has to sink more current than the second current source can supply. Therefore, the lacking current will be drawn from the inverting voltage mirror through the load, thereby supplying stimuli to the scalp or other body parts of the user. Thus, by controlling each current pump independently, such as to deliver stimuli to the user in a controlled manner, the disclosed invention has the advantage of delivering bidirectional current (including, but not limited to tACS, bipolar pulse stimulation, slow wave stimulation) without the necessity of using a negative voltage supply anywhere in the circuitry.

In an embodiment, the circuit includes a controller for controlling and processing one or more electric voltages and/or currents within the circuit arrangement for delivering brain stimuli to a user, a converter for converting digital signals to analogue signals within the circuit arrangement, and the pair of current sources to apply one or more stimuli to the electrodes of the circuit arrangement for delivering stimuli to the user.

Optionally, the first and the second current pumps collectively act as the source of the current performing brain/body stimulation and the inverting voltage mirror acts as the drain of the current performing brain/body stimulation.

Additionally, the first and the second current pumps collectively act as the drain of the current performing brain/body stimulation and the inverting voltage mirror acts as the source of the current performing brain/body stimulation.

Additionally, the stimulation circuit includes a monopolar voltage supply. More optionally, the monopolar voltage supply has a positive value.

Optionally, the circuit, when in use, includes components to measure and monitor the voltage and current applied across the load. In an exemplary embodiment (shown in Fig. 2b), such components include resistors, capacitors, diodes, transistors and operational amplifiers arranged so that the output from these components is directly proportional to the voltage or the current across the load. More optionally, such an output can be fed into analogue-to-digital converters for digitizing the output, as well as processing and storing measured voltage and current and for deriving values associated with an impedance of the load. Calculating, voltage, current, DC resistance and impedance is performed by standard methods known and available to those skilled in the art.

Furthermore, the stimulation circuit includes all, or a subset of, the aforementioned components all integrated inside a microchip. Such a microchip implementation is possible, because the circuit already includes operational amplifiers, diodes and transistors that are integrated-circuit-type components, as well as capacitors and resistors that are susceptible to being modelled with various technologies when designing such a microchip.

In an embodiment, the circuit may be used in a brain interfacing apparatus that provides simultaneous brain activity monitoring and stimulation of the brain of a user.

Throughout the present disclosure, the term "brain activity monitoring" relates to monitoring electrical signals received from a brain by a method including use of electroencephalography (EEG). Optionally, the brain activity monitoring may include detection of signals which include, but are not limited to, signals, or a combination of signals, obtained using electric field encephalography (EFEG), near infrared spectroscopy (NIRS), magnetoencephalography (MEG), electromyography (EMG) including signals generated from electrodes located spatially remotely from the given user's scalp, electrocardiography (ECG), eye tracking, functional magnetic resonance imaging (fMRI) and/or magnetic resonance imaging (MRI). More optionally, the brain activity monitoring relates to monitoring a change in electrical activity of the brain of a user, upon providing external electrical stimulus to the brain of the user. More optionally, the electrical activity of the brain of the user may be indicative of biological parameters related to the mental and physical health of the user including, but not limited to, a heart rate, a breathing rate and a skin conductance.

Optionally, the pair of electrodes may include separate electrodes configured for EEG recording and electrical stimulation respectively.

Alternatively, the pair of electrodes may include a separate electrode for each location at which it is desirable to detect EEG signals and/or provide electrical stimulation.

Optionally, the brain interfacing apparatus includes a data processing arrangement. Specifically, the data processing arrangement is configured to generate a brain stimulation using a protocol that is implemented using one or more adaptive learning algorithms or other computational algorithms after analysing the electrical signals received from the input/output arrangement. Specifically, the brain stimulation protocol may include, but is not limited to, using at least one of the following stimulation parameters: an amplitude, a phase, stimulation location, one or more frequencies with corresponding power for the brain stimuli to be generated, where these parameters are derived using one or more adaptive learning algorithms or other computational algorithms. Optionally, the brain stimulation protocol can give rise to brain stimuli in a form of a discrete signal or an arbitrary continuous waveform.

In an embodiment, the circuit, when in operation, may deliver electric stimuli to the user and simultaneously receive signals from skin of the user, at the point of contact of the electrodes.

### DETAILED DESCRIPTION OF DRAWINGS

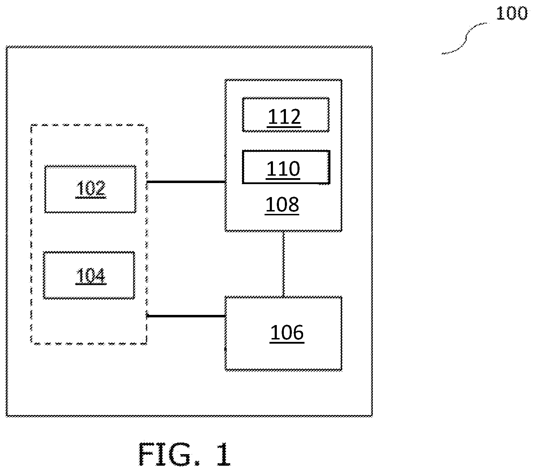

FIG. 1 is a block diagram of a circuit arrangement **100** for delivering electrical stimulation/stimuli to a user, in accordance with an embodiment of the present disclosure. The circuit arrangement **100** includes a pair of current sources including a first current source **102** and a second current source **104,** an inverting voltage mirror **106** and a load **108.** The pair of current sources **102** and **104** is configured using one or more components in the electronic circuit that delivers an electric current which is independent of a load across the circuit. The inverting voltage mirror **106** is a circuit that is designed to monitor and/or detect a change in voltage at a first end/node of the circuit and accordingly modulate the voltage at a second end/node of the circuit. The load **108** is a combination of the electrodes **110, 112** applied to a scalp and/or skin of other body parts of the user, any substances between the skin of the user and the electrodes and the body of the user. Alternatively, the load **108** may include invasive electrodes **110, 112** inserted into the body of the user and the body of the user.

FIG. 2a is a circuit diagram of a circuit **200** that is used in the circuit arrangement for delivering electrical stimulation/stimuli to a user, in accordance with an embodiment of the present disclosure. The circuit **200** includes a first current source **202,** a second current source **204** and an inverting voltage mirror **206.** Furthermore, the current sources **202, 204** and the inverting voltage mirror **206** are connected to a pair of electrodes **208** and **210,** which are connected to the body or concealed to the body, to constitute a load, particularly the load is constituted by a user's body, a pair of electrodes and any substances between the skin of the user and the electrodes. Specifically, the first current source **202,** the second source **204** are located at one side of the pair of electrodes **208, 210.** Moreover, the inverting voltage mirror **206** is configured to sample an output voltage of the current sources at the point **214** and to create corresponding inverted voltages on other side of the pair of electrodes **208, 210** (according to the above detailed description of operation of inverting voltage mirror) at a point **216.**

FIG. 2b is a circuit diagram of a circuit **200** used in the circuit arrangement for providing, namely delivering, electrical stimulation/stimuli to a user, in accordance with an embodiment of the present disclosure. The circuit **200** includes a first current source **202,** a second current source **204** and an inverting voltage mirror **206.** Furthermore, the current sources **202, 204** and the inverting voltage mirror **206** are connected to a pair of electrodes **208, 210,** which are connected to a body of the user or concealed to the body of the user, to constitute a load, wherein, in particular, the load is constituted by the user's body, a pair of electrodes and any substances between the skin of the user and the electrodes **208, 210.** Specifically, the first current source **202** and the second source **204** are located at one side of the pair of electrodes **208, 210,** respectively and the inverting voltage mirror **206** is configured to sample an output voltage of the current sources at the point **214** and creates a correspondingly inverted voltage on the other side of the pair of electrodes **208, 210** (according to the above detailed description of operation of inverting voltage mirror) at the point **216.** Furthermore, the first current source **202** includes a first input line **222** and the second current source **204** includes a second input line **220.** The first input line **222** and the second input line **220** may be connected to a microcontroller, a digital-to-analogue converter, or other sources of analogue signals (not shown in the figure). This circuitry also includes the components for measurement of current **231** and **232** pushed into the two electrodes and for measurement of voltages **233** and **234** at each electrode. The microcontroller, when in operation, may also be configured to control electric signals for the first current source **202** and the second current source **204,** as well as to receive information about the current and voltage from current measurement components **231** and **232** and from voltage measurement components **233** and **234.**

Conventionally, a single current source with positive and negative ("bipolar") voltage supply was utilized to generate inverting and noninverting output signals. Beneficially as compared to conventional approach, the circuit **200** of the present disclosure includes two separate and stable current sources, such as the first current source **202** and the second current source **204** so as to achieve the flow of current in the forward or the reverse direction. Moreover, the circuit **200** can be used to control each current source independently, such as to deliver stimuli to a user in a controlled manner, which can be useful in certain stimulation (e.g., related to cranial and other nerves), where a precise control over the current in each current source and bi-directional current delivery is required, while a bipolar voltage supply and/or bipolar current source is not practical. In addition, as the current sources are located on the same side of the load, therefore, the circuit **200** can be used to receive signals from skin of the user without any risk of electrical shock or other hazards to the user.

FIGs 3 and 4 are circuit diagrams of a first current source **300** (such as the first current source **202** of FIG. 2) and a second current source **400** (such as the second current source **204** of FIG. 2) present in the circuit (such as the circuit **200** of FIG. 2), in accordance with an embodiment of the present disclosure. The current sources **300** and **400** are electronic circuits that absorb or deliver an electric current which is independent of the output load. Furthermore, in use, one of the current sources **300** and **400** functions as a drain and the other one functions as a source. An output/input current of both current sources is controlled by an input signal **(302, 402),** for example a voltage signal, which may be supplied by a microcontroller, a digital-to-analogue converter, or other sources of analogue signals.

FIG. 5 is a circuit diagram of an inverting voltage mirror **500** (such as the inverting voltage mirror **206** of FIG. 2) present in the circuit of the circuit arrangement, in accordance with an embodiment of the present disclosure. Specifically, the inverting voltage mirror **500** monitors changes in voltage at a first end/node of the **circuit** (such as the point **214** of FIGs. 2a and 2b) and accordingly modulates the voltage at a second end/node of the circuit (such as the point **216** of FIGs. 2a and 2b).

FIGs. 6a and 6b are circuit diagrams of a voltage follower **602** and a voltage subtractor **604** present in the inverting voltage mirror (such as the inverting voltage mirror **206** of FIG. 2), according to an embodiment of the present disclosure. The voltage follower **602** is configured to monitor and/or detect a change in voltage at the first end/node of the circuit (such as the point **214** of FIGs. 2a and 2b). Furthermore, based on the change detected by the voltage follower **602,** the voltage subtractor **604** is configured to modulate the voltage at the second end/node of the circuit (such as the point **216** of FIGs. 2a and 2b).

FIG. 7 is a graphical representation of working of a conventionally used circuit in a stimulation arrangement. In the circuit used in conventional arrangements for delivering electrical stimuli to a user, one electrode has a fixed voltage value, while a voltage of the other electrode is varied. For example, an electrode on the right of the load is fixed to voltage V/2, and the voltage of the other electrode on the left of the load could be varied both up and down with respect to the value of voltage fixed on the right. This means that the current can flow in both directions, but a magnitude of the current is halved, as a maximum potential difference between the electrodes is +V/2 or -V/2. The circuit arrangement allows bidirectional current flow; however, since the maximum potential difference is limited to half of the voltage supplied by the power supply and the magnitude of the current is proportional to the potential difference, the magnitude of the maximum current that can be applied to stimulate the user is halved (compared to the maximum current that the given power supply can drive through the particular load).

FIG. 8 is a graphical representation of working of a circuit (such as the circuit **200** of FIG. 2) present in the circuit arrangement (such as the circuit arrangement **100** of FIG. 1), in accordance with an embodiment of the present disclosure. The potential of the left electrode is set by the two matched current sources. If one current source sources the same amount of current as the other one sinks, then the left electrode will be kept at voltage V/2 (where V is the maximum voltage of operation of the sourcing current source). The inverting voltage mirror of the circuit as used in the circuit arrangement **100,** is set to reference to the voltage V/2. It ensures that, when the potential of the left electrode is varied from V/2 in one direction (by creating source/sink imbalance of the two current pumps), the potential of the right electrode is displaced from V/2 by the same amount but in the opposite direction. Therefore, in a rest state (FIG. 8a), the left electrode is at V/2 and so the inverting voltage mirror automatically forces the right electrode to the same potential. Since both ends of the load are at the same potential, the current flow through the load is zero. Furthermore, when the imbalance between the sourced and sunk currents of the two current sources is introduced, the voltage at the left end of the load deviates from V/2. For example, if the voltage at the left end of the load is increased to a value 'a' above V/2, the inverting voltage mirror automatically moves the voltage at the right end of the load to the value 'a' below V/2 (FIG.8b), resulting in potential difference between the electrodes of 2a and current flowing from left to right. If the left end of the load is moved to 'b' below V/2, the inverting mirror will automatically move the right end to 'b' above V/2, resulting in potential difference across the load of 2b and the current flow from right to left. Again, in this set-up, the current flow is bi-directional, but the maximum potential difference between the electrodes is +V or -V, i.e., the same as the maximum voltage of operation of the sourcing current source (namely, very close to the maximum voltage of the power source).

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Reference to the singular is also to be construed to relate to the plural where appropriate.

Additional aspects, advantages, features and objects of the present disclosure would be made apparent from the drawings and the detailed description of the illustrative embodiments construed in conjunction with the appended claims that follow.

It will be appreciated that features of the present disclosure are susceptible to being combined in various combinations or replaced by other components without departing from the scope of the present disclosure as defined by the appended claims.

## Claims

1. A circuit arrangement (100) for delivering stimuli to a user, wherein the circuit arrangement comprises:
a circuit (200) including a pair of current pumps including a first current pump (102, 202, 300) and a second current pump (104, 204, 400), and
an inverting voltage mirror (106, 206, 500) to provide an inverted signal to at least one of the first current pump and a second current pump to control its operation, wherein the inverting voltage mirror is configured to maximize potential difference across nodes of the circuit.

2. The circuit arrangement (100) according to claim 1, wherein the first current pump (102, 202, 300) and the second current pump (104, 204, 400) are located on the same side of a load (108).

3. The circuit arrangement (100) according to any one of the preceding claims, wherein the circuit (200) is connected to a power source.

4. The circuit arrangement (100) according to claim 3, wherein the power source further includes at least one of a battery and a voltage boost circuit.

5. The circuit arrangement (100) according to any one of the preceding claims, wherein the inverting voltage mirror (106, 206, 500) is configured to ensure zero current flow when in its rest state.

6. The circuit arrangement (100) according to any one of the preceding claims, wherein the inverting voltage mirror (106, 206, 500) is located at the other side of the load (108) opposite to the first current pump (102, 202, 300) and the second current pump (104, 204, 400).

7. The circuit arrangement (100) according to any one of the preceding claims, wherein the circuit (200) is configured to create a potential difference across the load (108) required for supplying a predefined stimulus to the user.

8. The circuit arrangement (100) according to any one of the preceding claims, wherein the circuit arrangement (100) is configured to deliver at least one of a transcranial direct current stimulation (tDCS), transcranial alternating current stimulation (tACS) and bipolar pulse stimulation.

9. The circuit arrangement (100) according to any of the preceding claims, wherein the circuit (200), when in operation, delivers electric stimuli to the user and receives signals from skin of the user, simultaneously.

10. The circuit arrangement (100) according to any one of the preceding claims, wherein the circuit (200) includes a microprocessor configured to determine the parameters of stimulation.

11. The circuit arrangement (100) according to any of the preceding claims, wherein the circuit (200) includes components for measurement of current supplied to the electrodes.

12. The circuit arrangement (100) according to any of the preceding claims, wherein the circuit (200) includes components for measurement of voltages at the electrodes.

13. The circuit arrangement (100) according to any one of the preceding claims, wherein one or more components of the circuit (200) are arranged on an integrated circuit microchip.

## Patentansprüche

1. Schaltungsanordnung (100) zum Abgeben von Stimuli an einen Benutzer, wobei die Schaltungsanordnung Folgendes umfasst:
eine Schaltung (200), die ein Paar von Strompumpen umfasst, die eine erste Strompumpe (102, 202, 300) und eine zweite Strompumpe (104, 204, 400) umfassen, und
einen invertierenden Spannungsspiegel (106, 206, 500) zum Bereitstellen eines invertierten Signals für mindestens eine von der ersten Strompumpe und einer zweiten Strompumpe, um deren Betrieb zu steuern, wobei der invertierende Spannungsspiegel konfiguriert ist, eine Potentialdifferenz über Knoten der Schaltung zu maximieren.

2. Schaltungsanordnung (100) nach Anspruch 1, wobei die erste Strompumpe (102, 202, 300) und die zweite Strompumpe (104, 204, 400) auf derselben Seite einer Last (108) angeordnet sind.

3. Schaltungsanordnung (100) nach einem der vorhergehenden Ansprüche, wobei die Schaltung (200) mit einer Leistungsquelle verbunden ist.

4. Schaltungsanordnung (100) nach Anspruch 3, wobei die Leistungsquelle ferner mindestens eines von einer Batterie und einer Spannungserhöhungsschaltung umfasst.

5. Schaltungsanordnung (100) nach einem der vorhergehenden Ansprüche, wobei der invertierende Spannungsspiegel (106, 206, 500) konfiguriert ist, einen Nullstromfluss sicherzustellen, wenn er sich in seinem Ruhezustand befindet.

6. Schaltungsanordnung (100) nach einem der vorhergehenden Ansprüche, wobei der invertierende Spannungsspiegel (106, 206, 500) auf der anderen Seite der Last (108) gegenüber der ersten Strompumpe (102, 202, 300) und der zweiten Strompumpe (104, 204, 400) angeordnet ist.

7. Schaltungsanordnung (100) nach einem der vorhergehenden Ansprüche, wobei die Schaltung (200) konfiguriert ist, eine Potentialdifferenz über der Last (108) zu erzeugen, die zum Zuführen eines vordefinierten Stimulus an den Benutzer erforderlich ist.

8. Schaltungsanordnung (100) nach einem der vorhergehenden Ansprüche, wobei die Schaltungsanordnung (100) konfiguriert ist, mindestens eines von einer transkraniellen Gleichstromstimulation (tDCS), einer transkraniellen Wechselstromstimulation (tACS) und/oder einer bipolaren Pulsstimulation zu liefern.

9. Schaltungsanordnung (100) nach einem der vorhergehenden Ansprüche, wobei die Schaltung (200), wenn sie in Betrieb ist, elektrische Stimuli an den Benutzer abgibt und gleichzeitig Signale von der Haut des Benutzers empfängt.

10. Schaltungsanordnung (100) nach einem der vorhergehenden Ansprüche, wobei die Schaltung (200) einen Mikroprozessor umfasst, der konfiguriert ist, die Stimulationsparameter zu bestimmen.

11. Schaltungsanordnung (100) nach einem der vorhergehenden Ansprüche, wobei die Schaltung (200) Komponenten zur Messung des Stroms, der den Elektroden zugeführt wird, umfasst.

12. Schaltungsanordnung (100) nach einem der vorhergehenden Ansprüche, wobei die Schaltung (200) Komponenten zur Messung von Spannungen an den Elektroden umfasst.

13. Schaltungsanordnung (100) nach einem der vorhergehenden Ansprüche, wobei eine oder mehrere Komponenten der Schaltung (200) auf einem Mikrochip einer integrierten Schaltung angeordnet sind.

## Revendications

1. Agencement de circuit (100) pour délivrer des stimuli à un utilisateur, dans lequel l'agencement de circuit comprend :
un circuit (200) comprenant une paire de pompes de courant comprenant une première pompe de courant (102, 202, 300) et une deuxième pompe de courant (104, 204, 400), et
un miroir de tension d'inversion (106, 206, 500) pour fournir un signal inversé à l'au moins une de la première pompe de courant et d'une deuxième pompe de courant pour commander son fonctionnement, dans lequel le miroir de tension d'inversion est configuré pour maximiser la différence de potentiel entre les nœuds du circuit.

2. Agencement de circuit (100) selon la revendication 1, dans lequel la première pompe de courant (102, 202, 300) et la deuxième pompe de courant (104, 204, 400) sont situées sur le même côté d'une charge (108).

3. Agencement de circuit (100) selon l'une quelconque des revendications précédentes, dans lequel le circuit (200) est connecté à une source d'alimentation.

4. Agencement de circuit (100) selon la revendication 3, dans lequel la source d'alimentation comprend en outre l'au moins un d'une batterie et d'un circuit élévateur de tension.

5. Agencement de circuit (100) selon l'une quelconque des revendications précédentes, dans lequel le miroir de tension d'inversion (106, 206, 500) est configuré pour assurer une circulation de courant nulle lorsqu'il est dans son état de repos.

6. Agencement de circuit (100) selon l'une quelconque des revendications précédentes, dans lequel le miroir de tension d'inversion (106, 206, 500) est situé de l'autre côté de la charge (108) opposé à la première pompe de courant (102, 202, 300) et à la deuxième pompe de courant (104, 204, 400).

7. Agencement de circuit (100) selon l'une quelconque des revendications précédentes, dans lequel le circuit (200) est configuré pour créer une différence de potentiel aux bornes de la charge (108) requise pour fournir un stimulus prédéfini à l'utilisateur.

8. Agencement de circuit (100) selon l'une quelconque des revendications précédentes, dans lequel l'agencement de circuit (100) est configuré pour délivrer l'au moins une parmi une stimulation en courant continu transcrânien (tDCS), une stimulation en courant alternatif transcrânien (tACS) et une stimulation par impulsion bipolaire.

9. Agencement de circuit (100) selon l'une quelconque des revendications précédentes, dans lequel le circuit (200), lorsqu'il est en fonctionnement, délivre des stimuli électriques à l'utilisateur et reçoit des signaux de la peau de l'utilisateur, simultanément.

10. Agencement de circuit (100) selon l'une quelconque des revendications précédentes, dans lequel le circuit (200) comprend un microprocesseur configuré pour déterminer les paramètres de stimulation.

11. Agencement de circuit (100) selon l'une quelconque des revendications précédentes, dans lequel le circuit (200) comprend des composants pour la mesure du courant fourni aux électrodes.

12. Agencement de circuit (100) selon l'une quelconque des revendications précédentes, dans lequel le circuit (200) comprend des composants pour la mesure des tensions aux électrodes.

13. Agencement de circuit (100) selon l'une quelconque des revendications précédentes, dans lequel un ou plusieurs composants du circuit (200) sont agencés sur une micropuce de circuit intégré.
